# EUROPEAN PATENT SPECIFICATION

(11) **EP 4 683 616 B1**
(45) Date of publication and mention of the grant of the patent: **11.11.2026**
(21) Application number: 24713573.4
(22) Date of filing: 14.03.2024
(51) Int. Cl.: A61K 9/00, A61K 31/4168, A61K 47/18, A61K 47/32, A61K 47/38, A61P 27/04

(54) **PHARMACEUTICAL COMPOSITIONS COMPRISING APRACLONIDINE FOR OCULAR REDNESS RELIEF**
PHARMAZEUTISCHE ZUSAMMENSETZUNGEN MIT APRACLONIDIN ZUR LINDERUNG DER AUGENRÖTUNG
COMPOSITIONS PHARMACEUTIQUES COMPRENANT DE L'APRACLONIDINE POUR SOULAGER LA ROUGEUR OCULAIRE

(30) Priority: 18.03.2023 US 202363491051 P
(43) Date of publication of application: 28.01.2026
(73) Proprietor: Alcon Inc., 1701 Fribourg (CH)
(72) Inventor: RANGARAJAN, Rekha, Fort Worth, Texas 76134 (US); KETELSON, Howard Allen, Dallas, Texas 75204 (US); KOLEGA, Randall Renato, Fort Worth, Texas 76134 (US); BRIDLE, Mark Joseph, Fort Worth, Texas 76134 (US); MERZLIKINE, Alexei Grigoryevich, Fort Worth, Texas 76134 (US)
(74) Representative: Bohest AG
(86) International application number: PCT/IB2024/052504
(87) International publication number: WO 2024/194763

(56) References cited:
- WO-A1-2011/075617
- US-A1- 2012 309 804
- US-B2- 8 313 754

## Description

### Field

The present disclosure is directed to pharmaceutical compositions comprising apraclonidine and methods for reducing ocular redness in an eye of a subject by topically administering such compositions to the eye.

### Background

Ocular redness is a temporary condition that can result from minor irritations, such as being tired, oversleeping, allergic responses to environmental allergens, dry eyes, or irritation from wearing contact lenses. Current over-the-counter ophthalmic vasoconstrictors are not effective as long-term redness relievers. Available products in the market typically fall into the class of compounds referred to as "adrenergic agonists." Naphazoline hydrochloride, an α-1/α-2 mixed adrenergic agonist, and tetrahydrozoline, an α-1 selective adrenergic agent, bind to the adrenergic receptors on arterial vessels. Vasoconstriction of arteries in the short-term appear to provide redness relief. However, such vasoconstriction can produce several undesirable side effects such as rebound redness and tachyphylaxis in the long term. Selective targeting of blood vessels, specifically the venules, may help provide redness relief without the undesirable side effects, and appears to be the specific mode of action for α-2 adrenergic agonists.

Apraclonidine and brimonidine are both α-2 selective adrenergic agonists and have the same mode of action. Apraclonidine binds to adrenergic receptors in venules for capillary vasoconstriction, thereby providing adequate tissue oxygenation during product use. With adequate oxygenation, the side effect of rebound redness can be prevented as the drug effect wears-off. Other α-2 adrenergic agonists are not as prone to desensitization as the α-1 adrenergic agonists, thereby preventing tachyphylaxis. Apraclonidine is a hydrophilic analog of clonidine and reaches the target tissue, namely the conjunctiva, to provide direct redness relief. Moreover, apraclonidine and brimonidine have been previously used for anti-glaucoma management. The commercially available product IOPIDINE^{®} (apraclonidine ophthalmic solution) comprises apraclonidine at 0.5 % or 1 %. Given its use in ophthalmology and for glaucoma management at higher concentrations, apraclonidine has a well-established safety profile. However, known preparations of high concentrations of apraclonidine are complex and have intraocular pressure lowering effects and are therefore inappropriate for chronic use in ocular redness relief. Although no apraclonidine products are available for redness relief, a brimonidine product for this indication is currently commercially available from Bausch + Lomb and is sold as LUMIFY^{®} Brimonidine Tartrate Ophthalmic Solution 0.025%. The brimonidine in currently available products does not reach significant tissue levels in the conjunctiva, potentially a key target tissue for redness reduction. Without intending to be bound by any theory, a drug that reaches both the cornea and conjunctiva would be preferable because these are both key target tissues for redness relief. A drug that reaches both of these target tissues could therefore have a faster onset of action and/or longer duration of action.

U.S. Patent No. 9,259,425 ("Horn") reports pharmaceutical compositions with concentrations of selective α-2 adrenergic receptor agonists from about 0.0001% to about 0.05%. Moreover, Horn prefers brimonidine over apraclonidine and does not report experimental data for apraclonidine. Accordingly, there is a long felt but unsolved need for a pharmaceutical composition comprising an α-2 selective adrenergic agonist, wherein the α-2 selective adrenergic agonist has significant bioavailability in both the cornea and conjunctiva and the composition is effective for reducing ocular redness in an eye of a subject quickly (e.g., within 1 minute) and with a long duration of action (e.g., more than 6 - 8 hours).

### Summary

The present disclosure provides pharmaceutical compositions comprising apraclonidine, wherein apraclonidine is present at greater than 0.05% (w/v) to no more than 0.2% (w/v).

The present disclosure also provides pharmaceutical compositions comprising apraclonidine, wherein apraclonidine is present at greater than 0.05% (w/v) to no more than 0.2% (w/v), and wherein the pharmaceutical compositions comprise a polymer as a comfort and/or viscosity enhancer.

The present disclosure also provides pharmaceutical compositions comprising apraclonidine, wherein apraclonidine is present at greater than 0.05% (w/v) to no more than 0.2% (w/v), wherein apraclonidine exhibits higher bioavailability to a cornea than another pharmaceutical composition comprising brimonidine, wherein the brimonidine is present in the another pharmaceutical composition at 250 ppm.

The present disclosure also provides pharmaceutical compositions comprising apraclonidine, wherein apraclonidine is present at greater than 0.05% (w/v) to no more than 0.2% (w/v), wherein upon administration of the pharmaceutical compositions to an eye, apraclonidine exhibits higher bioavailability to a conjunctiva than another pharmaceutical composition comprising brimonidine, wherein the brimonidine is present in the another pharmaceutical composition at 250 ppm.

The present disclosure also provides methods of producing a pharmaceutical composition comprising apraclonidine, wherein apraclonidine is present at greater than 0.05% (w/v) to no more than 0.2% (w/v).

The present disclosure also provides methods of treating a subject having an ocular condition, such as red eye or irritation from dry eye or allergy, comprising administering a pharmaceutical composition comprising apraclonidine, wherein apraclonidine is present at greater than 0.05% (w/v) to no more than 0.2% (w/v). The pharmaceutical compositions comprise a polymer.

The present disclosure also provides methods of treating a subject having an ocular condition comprising administering a pharmaceutical composition comprising apraclonidine, wherein the apraclonidine is present at greater than 0.05% (w/v) to no more than 0.2% (w/v), wherein the apraclonidine exhibits higher bioavailability to a cornea than another pharmaceutical composition comprising brimonidine, wherein the brimonidine is present in the another pharmaceutical composition at 250 ppm.

The present disclosure also provides methods of treating a subject having an ocular condition or preventing a subject from developing an ocular condition comprising administering a pharmaceutical composition comprising apraclonidine, wherein the apraclonidine is present at greater than 0.05% (w/v) to no more than 0.2% (w/v), wherein the apraclonidine exhibits higher bioavailability to a conjunctiva than another pharmaceutical composition comprising brimonidine, wherein the brimonidine is present in the another pharmaceutical composition at 250 ppm.

### Brief Description Of The Drawings

The accompanying figures, which are incorporated in and constitute a part of this specification, illustrate several features of the present disclosure.

The patent or application file contains at least one drawing executed in color. Copies of this patent or patent application publication with color drawing(s) will be provided by the Office upon request and payment of the necessary fee.

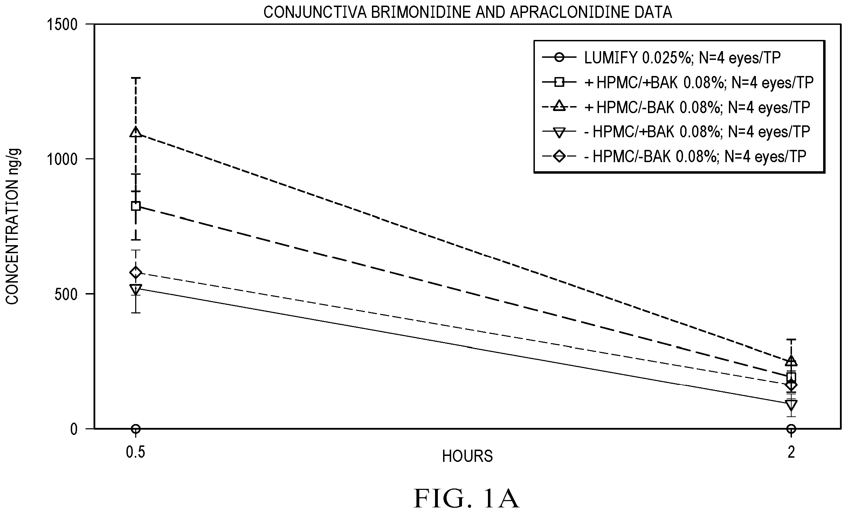

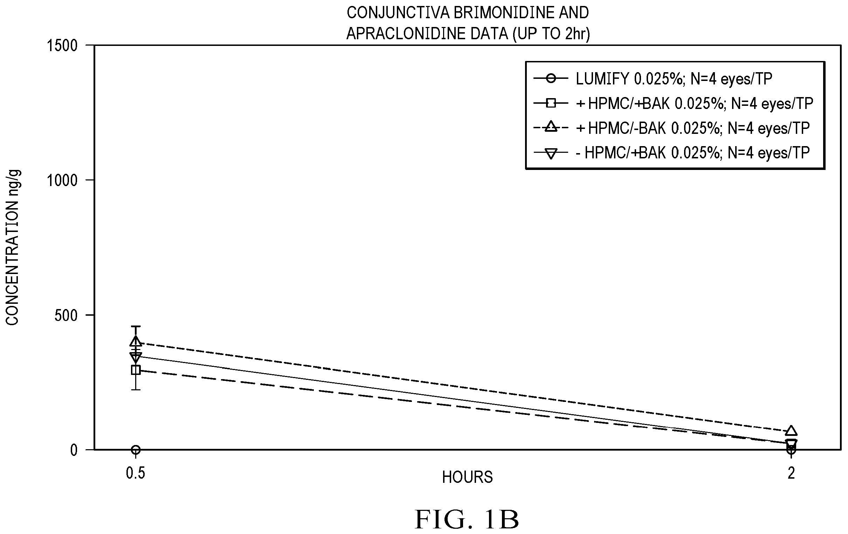

Figure 1A shows conjunctival bioavailability data for pharmaceutical compositions comprising 0.025% (w/w) brimonidine or 0.08% (w/v) apraclonidine thirty minutes and two hours after application to eyes. Figure 1B shows conjunctival bioavailability data for aqueous pharmaceutical compositions comprising 0.025% (w/w) brimonidine or 0.025% (w/v) apraclonidine thirty minutes and two hours after application to eyes. The ordinate represents concentration of brimonidine or apraclonidine in ng/g and the abscissa represents the time after administration. The circles represent data for LUMIFY^{®} (brimonidine tartrate); the squares represent data for +HPMC, +BAK apraclonidine compositions; the triangles represent data for +HPMC, -BAK apraclonidine compositions; the inverted triangles represent data for -HPMC, +BAK apraclonidine compositions; and the diamonds represent data for -HPMC, -BAK apraclonidine compositions. Four eyes were tested for each datum point.

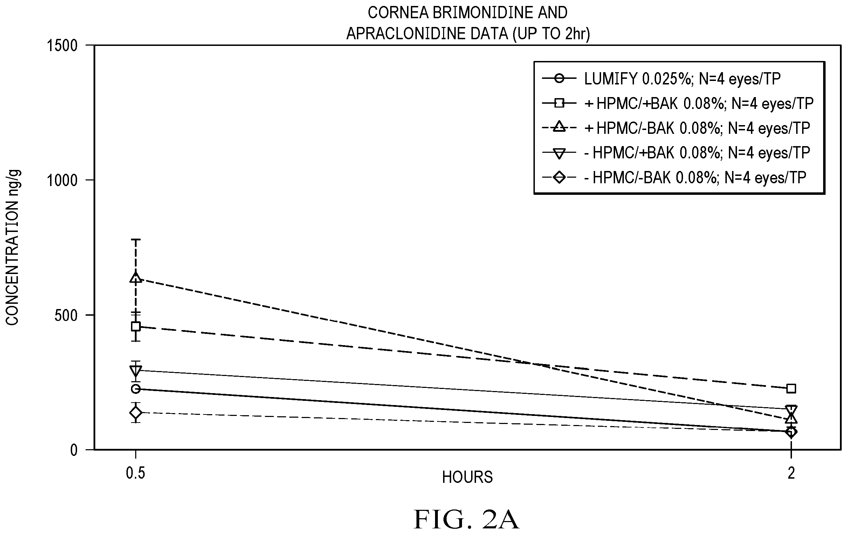

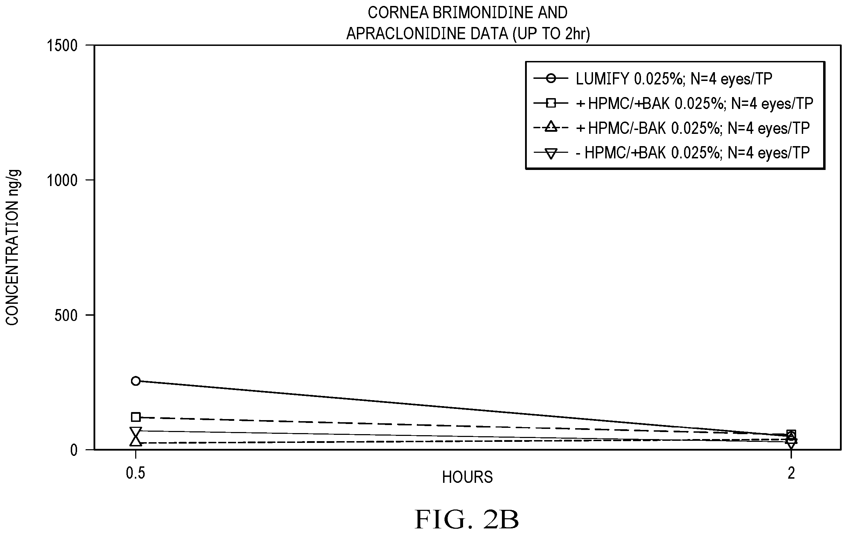

Figure 2A shows corneal bioavailability data for pharmaceutical compositions comprising 0.025% (w/w) brimonidine or 0.08% (w/v) apraclonidine thirty minutes and two hours after application to eyes. Figure 2B shows corneal bioavailability data for aqueous pharmaceutical compositions comprising 0.025% (w/w) brimonidine or 0.025% (w/v) apraclonidine thirty minutes and two hours after application to eyes. The ordinate represents concentration of brimonidine or apraclonidine in ng/g and the abscissa represents the time after administration. The circles represent data for LUMIFY^{®} (brimonidine tartrate); the squares represent data for +HPMC, +BAK apraclonidine compositions; the triangles represent data for +HPMC, -BAK apraclonidine compositions; the inverted triangles represent data for -HPMC, +BAK apraclonidine compositions; and the diamonds represent data for -HPMC, -BAK apraclonidine compositions. Four eyes were tested for each datum point.

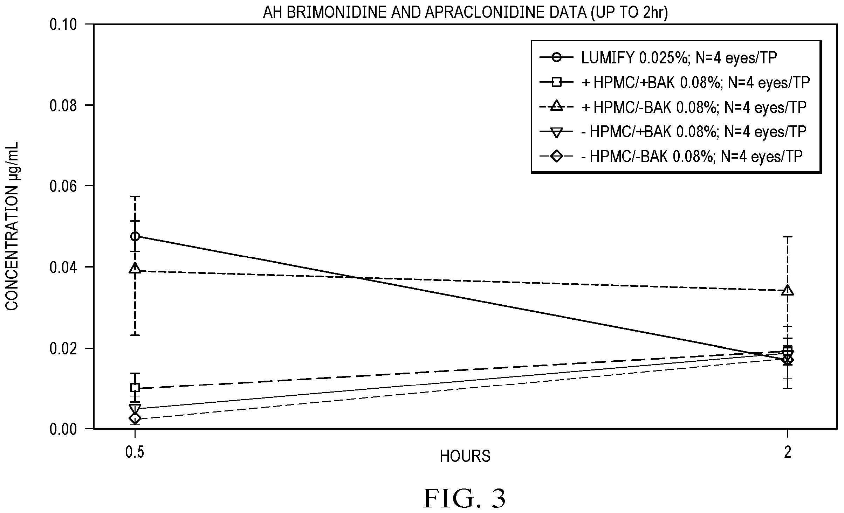

Figure 3 shows aqueous humor bioavailability data for pharmaceutical compositions comprising 0.025% (w/w) brimonidine or 0.08% (w/v) apraclonidine thirty minutes and two hours after application to eyes. The ordinate represents concentration of brimonidine or apraclonidine in ng/mL and the abscissa represents the time after administration. The circles represent data for LUMIFY^{®} (brimonidine tartrate); the squares represent data for +HPMC, +BAK apraclonidine compositions; the triangles represent data for +HPMC, -BAK apraclonidine compositions; the inverted triangles represent data for -HPMC, +BAK apraclonidine compositions; and the diamonds represent data for -HPMC, -BAK apraclonidine compositions. Four eyes were tested for each datum point.

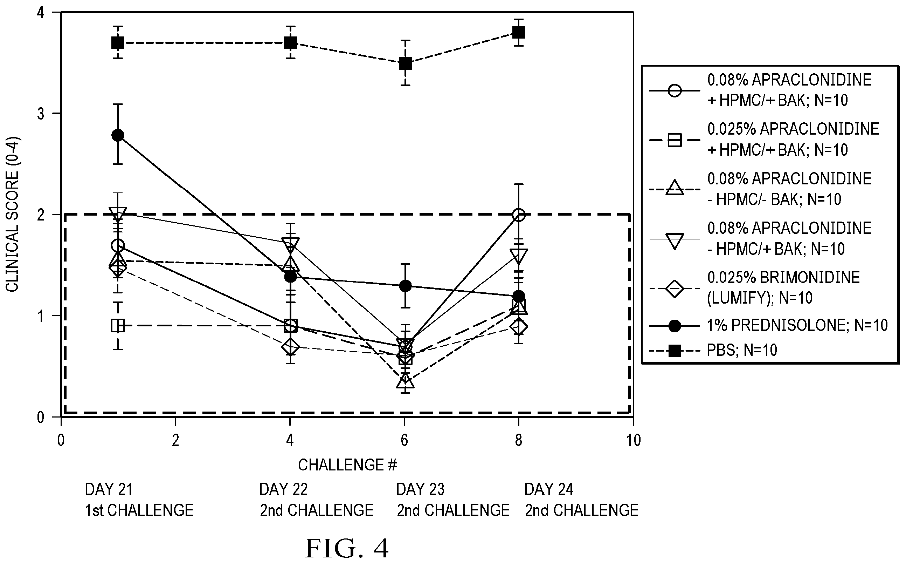

Figure 4 shows the ocular efficacy in a murine conjunctival allergen challenge ("CAC") model. The ordinate represents Clinical Score ranging from zero to four units while the abscissa represents the challenge number. The open circles represent data for +HPMC, +BAK pharmaceutical compositions comprising 0.08% (w/v) apraclonidine; the open squares represent data for +HPMC, +BAK pharmaceutical compositions comprising 0.025 %w/v apraclonidine; the triangles represent data for -HPMC, -BAK pharmaceutical compositions comprising 0.08% (w/v) apraclonidine; the inverted triangles represent data for -HPMC, -BAK pharmaceutical compositions comprising 0.08% (w/v) apraclonidine; the diamonds represent data for pharmaceutical compositions comprising 0.025% (w/w) brimonidine; the solid circles represent data for pharmaceutical compositions comprising 1% prednisone; and the solid squares represent data for phosphate-buffered saline. Ten eyes were examined for each datum point.

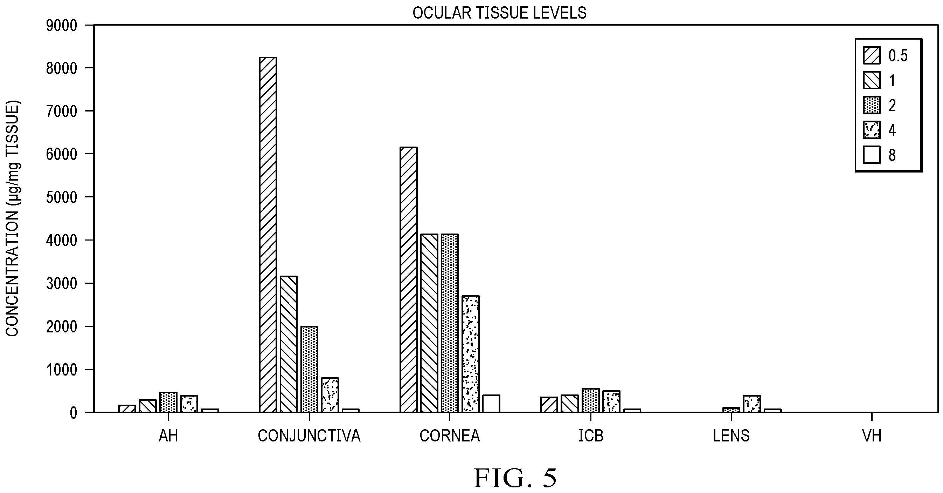

Figure 5 shows the ocular tissue distribution of a single topically administered 0.2% apraclonidine dose in rabbit eyes after 0.5, 1, 2, 4, and 8 hours.

### Description Of Embodiments

Unless defined otherwise, all technical and scientific terms have the same meaning as is commonly understood by one of ordinary skill in the art to which the disclosed embodiments belong.

As used herein, the terms "a" or "an" mean "at least one" or "one or more" unless the context clearly indicates otherwise.

As used herein, the term "animal" includes, but is not limited to, mammals, humans and non-human vertebrates, such as wild, domestic, and farm animals. **In** some embodiments, the animal being treated, such as a human, is "in need thereof." That is, the animal is in need of treatment.

As used herein, the terms "antagonize" and "antagonizing" mean reducing or completely eliminating one or more effects.

As used herein, the term "carrier" means a diluent, adjuvant, or excipient with which a compound is administered in a composition.

As used herein, the term, "compound" means all stereoisomers, tautomers, isotopes, and polymorphs of the compounds described herein.

As used herein, the terms "comprising" (and any form of comprising, such as "comprise", "comprises", and "comprised"), "having" (and any form of having, such as "have" and "has"), "including" (and any form of including, such as "includes" and "include"), or "containing" (and any form of containing, such as "contains" and "contain"), are inclusive and open-ended and include the options following the terms, and do not exclude additional, unrecited elements or method steps.

As used herein, the terms "individual," "subject," and "patient," used interchangeably, mean any animal described herein.

As used herein, the phrase "ophthalmically acceptable" means that the compounds, materials, compositions, and/or dosage forms are within the scope of sound medical judgment and are suitable for use in contact with the eyes of humans and other animals.

As used herein, the terms "treat," "treated," or "treating" mean both therapeutic treatment and prophylactic or preventative measures wherein the object is to prevent or slow down (lessen) an undesired physiological condition, disorder or disease, or obtain beneficial or desired clinical or veterinary results. For purposes herein, beneficial or desired clinical or veterinary results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state or remission (whether partial or total), whether detectable or undetectable; an amelioration of at least one measurable physical parameter, not necessarily discernible by the patient; or enhancement or improvement of condition, disorder or disease. Treatment includes eliciting a clinically or veterinarily significant response, optionally without excessive levels of side effects.

As used herein, the term "% (w/v)" means an amount (in grams) of a particular solute per 100 mL of solution.

As used herein, the term "% (w/w)" means the mass of a particular solute divided by the mass of the solution (solute and solvent together) and multiplied by 100 to obtain the percent.

The present disclosure provides pharmaceutical compositions suitable for topical administration to the eye and comprise apraclonidine, wherein the apraclonidine is present at greater than 0.05% (w/v) to no more than 0.2% (w/v). In some embodiments, the apraclonidine is present in the form of an ophthalmically acceptable salt. In a preferred embodiment, the pharmaceutical compositions comprise apraclonidine hydrochloride in an amount equivalent to an apraclonidine as free base concentration of greater than 0.05% (w/v) but not more than 0.2% (w/v). For example, if the apraclonidine is present in the form of apraclonidine hydrochloride, a composition comprising 0.125% (w/v) apraclonidine as a free base would contain 0.144% (w/v) apraclonidine hydrochloride.

The apraclonidine (as free base) is present in the pharmaceutical composition at greater than 0.05% (w/v) to no more than 0.2% (w/v). In some embodiments, the apraclonidine is present at 0.06% (w/v) to 0.2% (w/v). In some embodiments, the apraclonidine is present at 0.07% (w/v) to 0.15% (w/v). In some embodiments, the apraclonidine is present at 0.08% (w/v) to 0.125% (w/v). In one preferred embodiment, the apraclonidine is present at 0.06% (w/v). In another preferred embodiment, the apraclonidine is present at 0.08% (w/v). In a most preferred embodiment, the apraclonidine is present at 0.125% (w/v).

In some embodiments, the pharmaceutical composition is a liquid. In a preferred embodiment, the liquid pharmaceutical composition is an aqueous liquid.

In one embodiment, the pharmaceutical compositions comprising apraclonidine comprise an ophthalmically acceptable preservative. Several ophthalmically acceptable preservatives are known and include, for example, benzalkonium chloride and polyquaternium-1. In a preferred embodiment, the pharmaceutical compositions comprise benzalkonium chloride. In one embodiment, the compositions comprise benzalkonium chloride in an amount of 0.003 to 0.02% (w/v). In a most preferred embodiment, the compositions comprise 0.005% (w/v) benzalkonium chloride.

In other embodiments the pharmaceutical compositions comprising apraclonidine do not contain a preservative. In such embodiments, the compositions may be packaged in unit-dose containers or containers designed as multi-dose preservative-free (MDPF) containers. Unit-dose and multiple MDPF containers are known for topically administrable ophthalmic products, including prescription and over-the-counter (OTC) products.

The pharmaceutical compositions comprising apraclonidine comprise a polymer as a comfort and/or viscosity agent. In some embodiments, the polymer comprises methylcellulose, hydroxypropyl methylcellulose, hydroxyethyl-cellulose, carboxymethylcellulose, hydroxypropylcellulose, polyvinylpyrrolidone, polyvinyl alcohol, xanthan gum, carbopol, hyaluronic acid, guar or hydroxypropyl guar. The typical molecular weights for these polymers useful for viscosity enhancement range from 500K to 4 million. In a preferred embodiment, the compositions comprise hydroxypropyl methylcellulose as comfort and/or viscosity agent.

In some embodiments, the polymer is present at 0.05% to 0.5% (w/v). In some embodiments, the polymer is present at 0.2% to 0.4% (w/v). In some embodiments, the polymer is present at 0.3% (w/v). In a preferred embodiment, the compositions comprise 0.3% (w/v) hydroxypropyl methylcellulose
In some embodiments, the pharmaceutical compositions are liquids having a viscosity of 1 - 20 cP. In a preferred embodiment, the compositions have a viscosity of 5 to 15 cP. In a most preferred embodiment, the compositions have a viscosity of 6 to 8 cP.

In some embodiments, the pharmaceutical compositions comprising apraclonidine comprise a polymer that is a gelling agent in a concentration effective to promote gelling upon contact with the eye or with the tear film to remain in the eye for a prolonged period without loss by lacrimal drainage. Suitable gelling agents include, but are not limited to, thermosetting polymers such as tetra-substituted ethylene diamine block copolymers of ethylene oxide and propylene oxide (e.g., poloxamine 1307); polycarbophil; and polysaccharides such as gellan, carrageenan (e.g., kappa-carrageenan and iota-carrageenan), chitosan and alginate gums. Alternatively, guar based polymers may be utilized in the presence of borates to crosslink in the eye where the ocular pH enables the crosslinking to form guar gels.

In some embodiments, the pharmaceutical compositions comprising apraclonidine comprise a demulcent. Demulcents used with the compositions disclosed herein include, but are not limited to, glycerin, polyvinyl pyrrolidone, polyethylene oxide, polyethylene glycol, propylene glycol and polyacrylic acid. Preferred demulcents are propylene glycol and polyethylene glycol 400. In some embodiments, the polyethylene glycol is present at 0.4% (w/v).

The pharmaceutical compositions comprising apraclonidine are preferably buffered and comprise an ophthalmically acceptable buffer. In some embodiments, the ophthalmically acceptable buffer comprises a conjugate acid and a conjugate base. In some embodiments, the conjugate acid comprises acetic acid, boric acid, propionic acid, maleic acid, fumaric acid, lactic acid, malonic acid, malic acid, mandelic acid, citric acid, tartric acid, succinic acid, or phosphoric acid. In some embodiments, the conjugate base comprises an acetate, a propionate, a malate, a fumarate, a lactate, a malonate, a malate, a mandelate, a citrate, a tartrate, a succinate, or a phosphate. In a preferred embodiment, the compositions comprise a citrate buffer. In a most preferred embodiment, the compositions comprise citric acid and sodium citrate.

The pharmaceutical compositions comprising apraclonidine may comprise an ophthalmically acceptable pH-adjusting agent and are formulated at a pH in the range of 5.8 - 7.8. In a preferred embodiment, the compositions have a pH from 6.3 - 7.3. In a more preferred embodiment, the compositions have a pH from 6.5 - 7.0 and most preferably a pH of 6.8. Several ophthalmically acceptable pH-adjusting agents are known and include, for example, NaOH and HCl.

In some embodiments, the pharmaceutical compositions comprising apraclonidine are liquid compositions having an osmolarity of 260 to 330 mOsm/kg based on the total weight of the liquid pharmaceutical composition. In some embodiments, the liquid pharmaceutical composition has an osmolarity of 270 to 320 mOsm/kg based on the total weight of the liquid pharmaceutical composition. In some embodiments, the liquid pharmaceutical composition has an osmolarity of 280 to 310 mOsm/kg based on the total weight of the liquid pharmaceutical composition. In some embodiments, the liquid pharmaceutical composition has an osmolarity of 290 to 300 mOsm/kg based on the total weight of the liquid pharmaceutical composition. In some embodiments, the liquid pharmaceutical composition has an osmolarity of 300 mOsm/kg based on the total weight of the liquid pharmaceutical composition.

In some embodiments, the pharmaceutical compositions comprising apraclonidine comprise a tonicity adjusting agent. Many ophthalmically acceptable tonicity adjusting agents are known and include both ionic and nonionic agents. In some embodiments, the tonicity adjustment agent comprises sodium chloride. In some embodiments, the tonicity adjustment agent comprises sorbitol, propylene glycol, dextrose, glycerin, mannitol, or potassium chloride.

In some embodiments, the tonicity adjustment agent is present at the equivalent of 0.1%-0.8% (w/v) sodium chloride based on the total volume of the pharmaceutical composition. In a preferred embodiment, the pharmaceutical compositions comprise sodium chloride as a tonicity adjusting agent and the sodium chloride is present in an amount of 0.6 - 0.8% (w/v). In a most preferred embodiment, the composition comprises 0.7 - 0.8% (w/v) sodium chloride.

In some embodiments, the pharmaceutical compositions comprising apraclonidine can further comprise one or more antioxidants. Suitable antioxidants include, but are not limited to, ascorbic acid, sodium metabisulfite, sodium bisulfite, and acetylcysteine.

In some embodiments, the pharmaceutical compositions comprising apraclonidine can further comprise an anti-inflammatory agent. The anti-inflammatory agent can be steroidal or non-steroidal. Examples of suitable steroidal anti-inflammatory agents include, but are not limited to, dexamethasone, rimexolone, prednisolone, fluorometholone, and hydrocortisone. Examples of suitable non-steroidal anti-inflammatory agents include, but are not limited to, prostaglandin H synthetase inhibitors (Cox I or Cox II), also referred to as cyclooxygenase type I and type II inhibitors, such as diclofenac, flurbiprofen, ketorolac, suprofen, nepafenac, amfenac, indomethacin, naproxen, ibuprofen, bromfenac, ketoprofen, meclofenamate, piroxicam, sulindac, mefanamic acid, diflusinal, oxaprozin, tolmetin, fenoprofen, benoxaprofen, nabumetome, etodolac, phenylbutazone, aspirin, oxyphenbutazone, tenoxicam and carprofen; cyclooxygenase type II selective inhibitors, such as celecoxib and etodolac; PAF antagonists, such as apafant, bepafant, minopafant, nupafant and modipafant; PDE IV inhibitors, such as ariflo, torbafylline, rolipram, filaminast, piclamilast, cipamfylline, and roflumilast; and inhibitors of cytokine production, such as inhibitors of the NFkB transcription factor.

In some embodiments, the pharmaceutical compositions comprising apraclonidine can further comprise an anti-allergic agent. Examples of suitable anti-allergic agents include, but are not limited to, pemirolast, olopatadine, and corticosteroids (prednisolone, fluorometholone, loteprenol and dexamethasone).

The pharmaceutical compositions comprising apraclonidine can be in unit dosage form. In some embodiments, the unit dosage form is one or more containers filled with the compositions described herein. In such form, the pharmaceutical composition can be divided into unit doses containing appropriate quantities of the active component. The unit dosage form can be a packaged preparation. In some embodiments, the pharmaceutical compositions comprising apraclonidine are liquid compositions disposed in multidose packages that can support preserved and preservative-free packages. In some embodiments, the pharmaceutical compositions comprising apraclonidine are liquid compositions disposed in a single-use container. In some embodiments, the single-use container comprises a headspace. In some embodiments, the headspace is substantially free of oxygen. In some embodiments, the pharmaceutical compositions comprising apraclonidine are liquid compositions disposed in a multiple-use container. In some embodiments, the multiple-use container comprises a dropper bottle. In some embodiments, the multiple-use container comprises a bottle comprising a neck. In some embodiments, the pharmaceutical composition is contained within a single-dose non-reclosable container. Alternately, the pharmaceutical composition is contained within a multiple-dose reclosable container. In some embodiments, a desired dosage of the pharmaceutical composition comprising apraclonidine can be administered by means of a suitable dispenser as a known number of drops into the eye. Many examples of suitable multi-dose dropper bottle containers are known in the art and include, but are not limited to multi-dose dropper bottles such as the Droptainer^{™} bottle first introduced by Alcon and the Novelia^{®} multi-dose preservative-free eye dropper available from Nemera.

In some embodiments, the pharmaceutical compositions comprising apraclonidine are stable after storage for at least one month at 25°C. In some embodiments, the disclosed pharmaceutical compositions are stable after storage for at least one month at 40°C. In some embodiments, the pharmaceutical compositions comprising apraclonidine are stable after storage at 25°C/60% relative humidity (RH). In some embodiments, the pharmaceutical compositions comprising apraclonidine are stable after storage at 40°C/75% RH. In some embodiments, the stability can be measured by any one or more of appearance, container closure, pH, osmolality, viscosity, active assay value, the lack of impurities, and the like.

In some embodiments, upon administration of any of the pharmaceutical compositions comprising apraclonidine and lacking a preservative described herein to an eye, the apraclonidine in such a pharmaceutical composition exhibits higher bioavailability to a cornea compared to another pharmaceutical composition comprising brimonidine, wherein the brimonidine is present in the another pharmaceutical composition at 250 ppm.

In some embodiments, upon administration of any of the pharmaceutical compositions comprising apraclonidine and lacking a preservative described herein to an eye, the apraclonidine in such a pharmaceutical composition exhibits higher bioavailability to a conjunctiva compared to another pharmaceutical composition comprising brimonidine, wherein the brimonidine is present in the another pharmaceutical composition at 250 ppm.

In some embodiments, upon administration of any of the pharmaceutical compositions comprising apraclonidine described herein to an eye, such pharmaceutical compositions exhibit higher apraclonidine bioavailability to a cornea compared to the brimonidine bioavailability to a cornea in another pharmaceutical composition comprising 250 ppm brimonidine.

In some embodiments, upon administration of any of the pharmaceutical compositions comprising apraclonidine described herein to an eye, such pharmaceutical compositions exhibit higher apraclonidine bioavailability to a conjunctiva compared to the brimonidine bioavailability to a cornea in another pharmaceutical composition comprising 250 ppm brimonidine.

In any of these embodiments, the bioavailability can comprise corneal, conjunctival, or other ocular tissue bioavailability. In some embodiments, the bioavailability is assessed by pharmacokinetic methodology. For example, a dose administration can occur on Day 1, where one 40 µL drop of each test material can be administered to both eyes of each animal. A pipette can be used for precise administration. The time of dose administration and comments regarding the dose administration (such as, for example, good dose, if any was blinked out after administration, etc.) can be recorded in a study file. Sample collection can occur at, for example, 0.5, 2, 8, and 24 hours post dose administration. Animals can be euthanized (for example, n = 2 per group per time point). Eyes can be enucleated and the bulbar conjunctiva and corneas can be removed. For bulbar conjunctiva, samples of approximately 3 x 3 mm can be obtained from the inferior/nasal aspect of each eye (from, for example, the 4-6 o'clock position on the right eye and the 6-8 o'clock position on the left eye). Equipment can be wiped down and rinsed with saline and alcohol between each new eye. After removal, corneas and conjunctiva can be rinsed with saline, placed in a cryovial, and weighed. Cryovials can be weighed before and after tissues are placed inside and weights can be recorded. After the weights are recorded, the cryovials can be frozen on dry ice and kept on dry ice during sorting. Samples can be stored at -80°C until shipment.

In any of these embodiments, the another pharmaceutical compositions for comparison purposes can comprise a preservative-effective amount of a preservative. In any of these embodiments, the another pharmaceutical compositions for comparison purposes can comprise a preservative-effective amount of benzalkonium chloride. In some embodiments, the preservative-effective amount of the preservative is 80 ppm to 120 ppm, or 100 ppm. In some embodiments, the preservative-effective amount of benzalkonium chloride is 80 ppm to 120 ppm, or 100 ppm. In any of these embodiments, the another pharmaceutical compositions for comparison purposes is LUMIFY^{®} (see, world wide web at "accessdata.fda.gov/drugsatfda_does/nda/2017/208144Orig1s000PharmR.pdf at 7). In some embodiments, the 250 ppm brimonidine refers to 0.025% (w/w) brimonidine. In some embodiments, the brimonidine comprises brimonidine tartrate.

The present disclosure also provides methods of preparing the pharmaceutical compositions comprising apraclonidine described herein. In some embodiments, the apraclonidine is formulated in accordance with routine procedures as a pharmaceutical composition adapted for administration to humans. Typically, the pharmaceutical compositions are sterile isotonic aqueous buffered solutions. Where necessary, the pharmaceutical compositions can also include a solubilizing agent.

In some embodiments, the apraclonidine in any of the amounts disclosed herein is contacted with a liquid, such as an aqueous liquid. In some embodiments, the liquid or aqueous liquid comprises sodium chloride in a citrate or phosphate buffer. In some embodiments, benzalkonium chloride ("BAK") is added. In some embodiments, hydroxypropyl methylcellulose is added. In some embodiments, BAK and hydroxypropyl methylcellulose are added.

In brief, representative process steps by which apraclonidine hydrochloride ophthalmic solutions with the desired amount of apraclonidine hydrochloride can be prepared in the presence of a citrate buffer, sodium chloride, hydroxypropyl methylcellulose (HPMC), and benzalkonium chloride (BAK). The apraclonidine hydrochloride ophthalmic drug products can be prepared by mixing together the following in the specified amounts: 6 mg/g HPMC stock; and 2) 4.1 mg/g apraclonidine hydrochloride with a 43 mM sodium citrate, 0.9 mM citric acid buffer, with sodium chloride added as a tonicity adjuster and 0.14 mg/g BAK. For example, a 1.44 mg/g apraclonidine hydrochloride ophthalmic drug product can be prepared by steam sterilizing 6 mg/g HPMC stock, the apraclonidine hydrochloride buffered solution may be sterile filtered with a 0.2 µm PVDF filter and added to the HPMC stock. The resulting solution may be adjusted to final pH and adjusted to batch size with sterile purified water.

The present disclosure also provides methods of treating a subject having ocular redness, the methods comprising administering to the subject any of the pharmaceutical compositions comprising apraclonidine described herein to an affected eye.

In some embodiments, the ocular condition is hyperemia, ocular redness, dry eye, or an allergic response. In some embodiments, the ocular condition is hyperemia. In some embodiments, the ocular condition is ocular redness such as redness caused by minor irritation. In some embodiments, the ocular condition is dry eye. In some embodiments, the ocular condition is an allergic response.

In some embodiments, the administration is to a single eye of a subject. In some embodiments, the administration is to both eyes of a subject.

The present disclosure also provides methods of reducing ocular redness in an eye of a subject, the methods comprising administering to the subject any of the pharmaceutical compositions comprising apraclonidine described herein to an eye.

In any of the treatment methods disclosed herein, upon administration of any of the pharmaceutical compositions comprising apraclonidine and a polymer described herein to an eye, the apraclonidine in such a pharmaceutical composition exhibits higher bioavailability to a cornea compared to another pharmaceutical composition comprising brimonidine, wherein the brimonidine is present in the another pharmaceutical composition at 250 ppm.

In any of the treatment methods disclosed herein, upon administration of any of the pharmaceutical compositions comprising apraclonidine and a polymer described herein to an eye, the apraclonidine in such a pharmaceutical composition exhibits higher bioavailability to a conjunctiva compared to another pharmaceutical composition comprising brimonidine, wherein the brimonidine is present in the another pharmaceutical composition at 250 ppm.

In any of these embodiments, the bioavailability can comprise corneal, conjunctival, or ocular bioavailability. In some embodiments, the bioavailability is assessed by methodology described herein.

In any of these embodiments, the another pharmaceutical compositions for comparison purposes can comprise a preservative-effective amount of a preservative. In any of these embodiments, the another pharmaceutical compositions for comparison purposes can comprise a preservative-effective amount of benzalkonium chloride. In some embodiments, the preservative-effective amount of the preservative is 80 ppm to 120 ppm, or 100 ppm. In some embodiments, the preservative-effective amount of benzalkonium chloride is 80 ppm to 120 ppm, or 100 ppm. In any of these embodiments, the another pharmaceutical compositions for comparison purposes is LUMIFY^{®} (see, world wide web at "accessdata.fda.gov/drugsatfda_does/nda/2017/208144Orig1s000PharmR.pdf at 7). In some embodiments, the 250 ppm brimonidine refers to 0.025% (w/w) brimonidine. In some embodiments, the brimonidine comprises brimonidine tartrate.

In any of the treatment methods disclosed herein, the pharmaceutical compositions comprising apraclonidine can be administered to the eye in the form of an eye drop.

In some embodiments, the pharmaceutical compositions comprising apraclonidine are administered topically to one or more tissues of the eye. In one embodiment, the compositions are administered topically to an eye from one to four times per day. In a preferred embodiment, the compositions are administered topically from one to two times per day. In a more preferred embodiment where the compositions of the present invention comprise 0.125% (w/v) of apraclonidine, the compositions are effective in reducing redness within 1 minute following topical administration and are effective in sustaining a reduction in redness for 8 hours or more. In a most preferred embodiment, the compositions are effective in reducing redness within 1 minute following topical administration and are effective in sustaining a reduction in redness for 10 hours or more.

The present disclosure also provides use of any of the pharmaceutical compositions comprising apraclonidine described herein in the manufacture of a medicament for reducing ocular redness caused by minor irritations. In some embodiments, the ocular redness is hyperemia or ocular redness caused by dry eye or an allergic response.

In order that the subject matter disclosed herein may be more efficiently understood, examples are provided below. It should be understood that these examples are for illustrative purposes only and are not to be construed as limiting the claimed subject matter in any manner.

### Examples

### Example 1: Preparation of Phosphate Buffered 0.08% (w/v) and 0.025% (w/v) Apraclonidine Compositions

### 800 ppm and 250 ppm Drug Product Formulations

Representative 800 ppm and 250 ppm drug product formulations are presented in Table 1 and Table 2, respectively.

**Table 1: 800 ppm Apraclonidine**

| Ingredient | Concentration (mg/g) |
|---|---|
| Apraclonidine (free base) | 0.8 |
| Dihydrogen phosphate monohydrate | 0.93 |
| Disodium hydrogen phosphate heptahydrate | 3.6 |
| Sodium Chloride | 7.7 |
| Hydroxypropyl Methylcellulose | 4.8 |
| Benzalkonium Chloride | 0.1 |
| WFI | qs |

**Table 2: 250 ppm Apraclonidine**

| Ingredient | Concentration (mg/g) |
|---|---|
| Apraclonidine (free base) | 0.25 |
| Dihydrogen phosphate monohydrate | 0.87 |
| Disodium hydrogen phosphate heptahydrate | 3.3 |
| Sodium Chloride | 7.7 |
| Hydroxypropyl Methylcellulose | 4.8 |
| Benzalkonium Chloride | 0.1 |
| WFI | qs |

The drug products were prepared by mixing the stock solutions as described below. The appropriate stock solutions can be added in the following order: 1) apraclonidine stock with sodium chloride, 2) HPMC stock, 3) sterile filtered BAK stock, and 4) sterile filtered WFI water, if necessary.

**Table 3: Preparation of 250 ppm Drug Product with Stock Solutions**

| Solution | Weight, % |
|---|---|
| Apraclonidine stock with sodium chloride (0.6 mg/g APR stock in phosphate buffer) | 42% |
| HPMC stock | 48% |
| BAK stock (not added until after filtration of drug product) | 10% |
| WFI water | QS to 100% |

**Table 4: Preparation of 800 ppm Drug Product with Stock Solutions**

| Solution | Weight, % |
|---|---|
| Apraclonidine stock with sodium chloride (1.92 mg/g APR stock in phosphate buffer) | 41.6% |
| HPMC stock | 48% |
| BAK stock (not added until after filtration of drug product) | 10% |
| WFI water | QS to 100% |

The drug product was gently mixed with an overhead mixer with the 1.5 in stainless steel mixing paddle for 15-20 minutes to ensure uniformity. In the laminar flow hood, the drug product containing the apraclonidine stock with sodium chloride and HPMC stock was sterile filtered with a 0.2 µm PVDF membrane into an appropriately sized sterile Nalgene bottle. Following filtration, the required amount of sterile filtered BAK stock was added and the capped Nalgene bottle was gently inverted to mix. Sterile filtered WFI water was added to bring the formulation to the target volume.

### Example 2: Stability of 0.08% (w/v) Apraclonidine Composition of Example 1

Aqueous pharmaceutical compositions comprising 0.08% (w/v) apraclonidine (free base), 7.66 mg/g sodium chloride in a phosphate buffer were prepared. 0.10 mg/g of benzalkonium chloride ("BAK") and/or 4.84 mg of hydroxypropyl methylcellulose 2910 ("HPMC") was added. This resulted in pharmaceutical compositions: i) without BAK or HPMC ("-HPMC, -BAK"); ii) with BAK but without HPMC ("-HPMC, +BAK"); iii) without BAK but with HPMC ("+HPMC, -BAK"); and iv) with BAK and HPMC ("+HPMC, +BAK"). Portions of compositions i)-iv) comprising 0.08% (w/v) apraclonidine were incubated for four weeks at 25°C ("T4, 25C"), for four weeks at 40°C ("T4, 40C"), or not incubated "T0"). Stability data for the four pharmaceutical compositions were then determined. The appearance, level of apraclonidine, level of total impurities, level of BAK, pH value, osmolarity value, and viscosity value for each of the four pharmaceutical compositions were found to be maintained upon storage for four weeks at 25°C or 40°C. The results are shown in Tables 5a - 5d below.

**Table 5a: -HPMC, -BAK**

| Tests | Target Spec | T0 | T4 25C | T4 40C |
|---|---|---|---|---|
| Appearance | Clear, Colorless Liquid | Conforms | Conforms | Conforms |
| API Assay (mg/g) | 0.76 - 0.84 (95-105% LC) | 0.811 (101.3% LC) | 0.804 (100.5% LC) | 0.807 (100.8% LC) |
| Related Substances | < 1.0% Total Impurities | 0.0% | 0.0% | 0.0% |
| BAK Assay (mg/g) | 0.08 - 0.12 (80-120% LC) | Not tested | Not tested | Not tested |
| pH | 7.0 (6.8 - 7.2) | 7.07 | 7.147 | 7.105 |
| Osmolality (mOsm/Kg) | 300 (280 - 320) | 287 | 290 | 288 |
| Viscosity (cPs) | 19 (17-21) | Not tested | Not tested | Not tested |

**Table 5b: -HPMC, +BAK**

| Tests | Target Spec | T0 | T4 25C | T4 40C |
|---|---|---|---|---|
| Appearance | Clear, Colorless Liquid | Conforms | Conforms | Conforms |
| API Assay (mg/g) | 0.76 - 0.84 (95-105% LC) | 0.826 (103.3% LC) | 0.809 (101.1% LC) | 0.808 (101% LC) |
| Related Substances | < 1.0% Total Impurities | 0.0% | 0.0% | 0.0% |
| BAK Assay (mg/g) | 0.08 - 0.12 (80-120% LC) | 0.094 (94% LC) | 0.093 (93% LC) | 0.095 (95% LC) |
| pH | 7.0 (6.8 - 7.2) | 7.00 | 7.119 | 7.105 |
| Osmolality (mOsm/Kg) | 300 (280 - 320) | 287 | 294 | 288 |
| Viscosity (cPs) | 19 (17-21) | Not tested | Not tested | Not tested |

**Table 5c: +HPMC, -BAK**

| Tests | Target Spec | T0 | T4 25C | T4 40C |
|---|---|---|---|---|
| Appearance | Clear, Colorless Liquid | Conforms | Conforms | Conforms |
| API Assay (mg/g) | 0.76 - 0.84 (95-105% LC) | 0.829 (103.6% LC) | 0.817 (102.1% LC) | 0.815 (101.9% LC) |
| Related Substances | < 1.0% Total Impurities | 0.0% | 0.0% | 0.0% |
| BAK Assay (mg/g) | 0.08 - 0.12 (80-120% LC) | Not tested | Not tested | Not tested |
| pH | 7.0 (6.8 - 7.2) | 7.05 | 7.107 | 7.094 |
| Osmolality (mOsm/Kg) | 300 (280 - 320) | 296 | 299 | 295 |
| Viscosity (cPs) | 19 (17-21) | 19.3 | 17.9 | 17.2 |

**Table 5d: +HPMC, +BAK**

| Tests | Target Spec | T0 | T4 25C | T4 40C |
|---|---|---|---|---|
| Appearance | Clear, Colorless Liquid | Conforms | Conforms | Conforms |
| API Assay (mg/g) | 0.76 - 0.84 (95-105% LC) | 0.829 (103.6% LC) | 0.823 (102.8% LC) | 0.830 (103.7% LC) |
| Related Substances | < 1.0% Total Impurities | 0.0% | 0.0% | 0.0% |
| BAK Assay (mg/g) | 0.08 - 0.12 (80-120% LC) | 0.094 (94% LC) | 0.095 (95% LC) | 0.095 (95% LC) |
| pH | 7.0 (6.8 - 7.2) | 6.99 | 7.097 | 7.101 |
| Osmolality (mOsm/Kg) | 300 (280 - 320) | 301 | 302 | 301 |
| Viscosity (cPs) | 19 (17-21) | 18.9 | 18.0 | 17.3 |

### Example 3: 0.08% (w/v) Apraclonidine Low Salt Drug Product Composition

A Representative 800 ppm low salt drug product formulations is presented in Table 6.

**Table 6: 800 ppm Low Salt**

| Ingredient | Concentration (mg/g) |
|---|---|
| Apraclonidine (free base) | 0.8 |
| Dihydrogen phosphate monohydrate | 0.93 |
| Disodium hydrogen phosphate heptahydrate | 3.6 |
| Sodium Chloride | 6.0 |
| Glycerol | 4.5 |
| Hydroxypropyl Methylcellulose | 3.0 |
| Benzalkonium Chloride | 0.1 |
| WFI | QS |

The drug product was prepared by mixing the stock solutions as described below. The appropriate stock solutions can be added in the following order: 1) apraclonidine stock, 2) 49.05 mM phosphate buffer, salt, glycerol stock, 3) HPMC stock, 4) sterile filtered BAK stock, and 5) sterile filtered WFI water, if necessary.

**Table 7: Preparation of 800 ppm Drug Product with Stock Solutions**

| Solution | Weight, % |
|---|---|
| 49.05 mM phosphate buffer with sodium chloride and glycerol | 40% |
| Apraclonidine stock (4 mg/g) | 20% |
| HPMC stock (10 mg/g) | 30% |
| BAK stock (1 mg/g) (not added until after filtration of drug product) | 10% |
| WFI water | QS to 100% |

The drug product was gently mixed with an overhead mixer with the 1.5 in stainless steel mixing paddle for 15-20 minutes to ensure uniformity. In the laminar flow hood, the drug product containing the apraclonidine stock, the 49.05 mM phosphate buffer with sodium chloride and glycerol, and HPMC stock was sterile filtered with a 0.2 µm PVDF membrane into an appropriately sized sterile Nalgene bottle. Following filtration, the required amount of sterile filtered BAK stock was added and the capped Nalgene bottle was gently inverted to mix. Sterile filtered WFI water was added to bring the formulation to the target volume.

### Example 4: Ocular Bioavailability of Aqueous Pharmaceutical Compositions of Example 1

LUMIFY^{®} (a pharmaceutical composition comprising 0.025% (w/w) brimonidine tartrate) was obtained. Additionally, the -HPMC/-BAK, -HPMC/+BAK, +HPMC/-BAK, and +HPMC/+BAK pharmaceutical compositions comprising 0.08 (w/v) apraclonidine and the - HPMC/+BAK, +HPMC/-BAK, and +HPMC/+BAK pharmaceutical compositions comprising 0.025% (w/v) apraclonidine of Example 1 were obtained. The conjunctival and corneal bioavailability for the pharmaceutical compositions comprising brimonidine and apraclonidine were determined in rabbits. In particular, the ocular tissues were thawed and homogenized in HPLC grade water. Homogenates were analyzed using HPLC and pre-established bioanalytical method for apraclonidine. Data for four eyes at thirty minutes or two hours after administration were obtained.

The LUMIFY^{®} pharmaceutical composition comprising 0.025% (w/w) brimonidine was found to have negligible conjunctival bioavailability thirty minutes and two hours after application to eyes. In contrast, the -HPMC/-BAK, -HPMC/+BAK, +HPMC/-BAK, and +HPMC/+BAK pharmaceutical compositions comprising 0.08% (w/v) apraclonidine were found to have good conjunctival bioavailability with pharmaceutical compositions comprising HPMC having excellent conjunctival bioavailability (see, Figure 1A). In Figure 1A, at 30 minutes, statistically higher levels of apraclonidine were observed in the conjunctiva relative to brimonidine for all tested formulations (p< 0.0001). Statistically significant improvements in bioavailability were observed for the +HPMC, -BAK apraclonidine formulation relative to the - HPMC,-BAK and -HPMC,+BAK containing formulations (p< 0.0001). The +HPMC,-BAK formulation showed a statistically significant improvement in conjunctival bioavailability relative to the +HPMC,+BAK formulation (p≤ 0.05). Given that brimonidine and apraclonidine are both α-2 selective adrenergic agonists, similar bioavailability might have been expected. Therefore, the results obtained for the -HPMC/-BAK, -HPMC/+BAK, +HPMC/-BAK, and +HPMC/+BAK pharmaceutical compositions comprising 0.08% (w/v) apraclonidine were unexpected. The finding that pharmaceutical compositions comprising HPMC had better conjunctival bioavailability was similarly unexpected. In contrast, BAK did not have a great effect on conjunctival bioavailability.

While the conjunctival bioavailability in -HPMC/+BAK, +HPMC/-BAK, and +HPMC/+BAK pharmaceutical compositions comprising 0.025% (w/v) apraclonidine was less than that for comparable pharmaceutical compositions comprising 0.08% (w/v) apraclonidine, such bioavailability was unexpectedly greater than that of the LUMIFY^{®} pharmaceutical composition (see, Figure 1B). In Figure 1B, at 30 minutes statistically higher levels of apraclonidine was observed in the conjunctiva with all formulations relative to LUMIFY (p< 0.0001). No difference in bioavailability was observed amongst the apraclonidine containing formulations (ns). These data show that the increased conjunctival bioavailability for pharmaceutical compositions comprising 0.08% (w/v) apraclonidine with respect to the LUMIFY^{®} pharmaceutical composition comprising 0.025% (w/w) brimonidine did not simply stem from a greater amount of α-2 selective adrenergic agonist. Thus, pharmaceutical compositions comprising greater than 0.05% (w/v) to no more than 0.2% (w/v) apraclonidine would exhibit unexpectedly good conjunctival bioavailability.

While the corneal bioavailability of the LUMIFY^{®} pharmaceutical composition comprising 0.025% (w/w) brimonidine after thirty minutes and two hours was comparable to - HPMC/-BAK and -HPMC/+BAK pharmaceutical compositions comprising 0.08% (w/v) apraclonidine, the corneal bioavailability of the +HPMC/-BAK and +HPMC/+BAK pharmaceutical compositions comprising 0.08% (w/v) apraclonidine were associated with excellent corneal bioavailability (see, Figure 2A). In Figure 2A, at 30 minutes, +HPMC,-BAK (p< 0.0001) and +HPMC, +BAK (p< 0.01) apraclonidine formulations demonstrated statistically higher levels of the active in the cornea vs. LUMIFY. The +HPMC,-BAK apraclonidine formulation also showed statistically higher levels of apraclonidine in the cornea relative to - HPMC,+BAK and -HPMC,-BAK (p< 0.0001). The +HPMC,-BAK formulation showed a statistically significant improvement in conjunctival bioavailability relative to the +HPMC,+BAK formulation (p≤ 0.05). Surprisingly, the corneal bioavailability of the LUMIFY^{®} pharmaceutical composition comprising 0.025% (w/w) brimonidine was found to be greater than that of any of the pharmaceutical compositions comprising 0.025% (w/v) apraclonidine tested (see, Figure 2B). In Figure 2B, at 30 minutes a statistically higher level of brimonidine was observed in the cornea relative to all apraclonidine containing formulations (p< 0.0001). A difference in bioavailability was observed between +HPMC,+BAK relative to +HPMC,-BAK (p< 0.01), while no difference in bioavailability was observed between +HPMC,+BAK and - HPMC,+BAK (ns).

The aqueous humor bioavailability of the LUMIFY^{®} pharmaceutical composition comprising 0.025% (w/w) brimonidine thirty minutes after administration was found to be greater than that for the pharmaceutical compositions comprising 0.08% (w/v) apraclonidine. Surprisingly, however, the aqueous humor bioavailability of the +HPMC/-BAK pharmaceutical composition was maintained two hours of administration while that of the -HPMC/-BAK, -HPMC/+BAK, and +HPMC/+BAK pharmaceutical compositions was increased two hours after administration. In contrast, the aqueous humor bioavailability of the LUMIFY^{®} pharmaceutical composition decreased two hours after administration (see, Figure 3). At 30 minutes, there was no statistically significant difference in aqueous humor levels of brimonidine relative to apraclonidine delivered in a +HPMC,-BAK formulation (ns). The -HPMC,-BAK and - HPMC, +BAK formulations showed statistically lower aqueous humor levels of apraclonidine relative to +HPMC,-BAK formulation (p< 0.001). The +HPMC,-BAK formulation showed an improvement in aqueous humor bioavailability relative to the +HPMC,+BAK formulation (p<0.01).

### Example 5: Efficacy of Apraclonidine Compositions of Example 1

Ocular efficacy was examined in a murine CAC model. On day 0, sensitization to short ragweed ("SRW") antigen was induced in mice by subcutaneous injections of SRW in Freund's adjuvant. The animals were challenged with topical SRW on day 18 and then randomized based on their overall response to the challenge. On days 19 to 20, the mice were administered pharmaceutical formulations or phosphate-buffered saline. On day 21, the mice were again administered the pharmaceutical formulations or phosphate-buffered saline. Twenty minutes later, the mice were evaluated as described below. Thirty to 35 minutes after the administration of the pharmaceutical formulations or phosphate-buffered saline, the mice were administered topical SRW antigen. The mice were again evaluated eighteen minutes after administration of topical SRW antigen ("Challenge 1"). Topical SRW antigen was administered later on day 21. On days 22 to 24, the pharmaceutical formulations or phosphate-buffered saline was twice administered followed by administration of the topical SRW antigen as described for day 21. Mice were evaluated during and after the second round of administration on days 22 to 24 as described for day 21 ("Challenges 4, 6, and 8," respectively). The evaluations comprised scoring for squinting, hyperemia, discharge, and lid swelling. Scoring was on a scale of "0" to "4" with a score of "0" representing a "normal" eye while a score of "4" represented "severe." Squinting was assessed grossly while hyperemia, discharge, and lid swelling was assessed by a masked examiner under a Micron III imaging system. Only the right eye was evaluated. The score for each criteria was added and averaged to provide a "Clinical Score" ranging from zero to four with a Clinical Score of zero to one representing a gold standard.

Eyes to which phosphate-buffered saline was administered before administration of the SRW antigen had a greater Clinical Score than eyes administered pharmaceutical compositions comprising 0.025% (w/v) apraclonidine, 0.08% (w/v) apraclonidine, 0.025% (w/w) brimonidine, or 1% (w/v) prednisone. The Clinical Score for each of: i) +HPMC/+BAK pharmaceutical compositions comprising 0.08% (w/v) apraclonidine; ii) +HPMC/+BAK pharmaceutical compositions comprising 0.025% (w/v) apraclonidine; iii)
- HPMC/-BAK pharmaceutical compositions comprising 0.08% (w/v) apraclonidine; and iv)
- HPMC/+BAK pharmaceutical compositions comprising 0.08% (w/v) apraclonidine were similar to that of pharmaceutical compositions comprising 0.025% (w/w) brimonidine (see, Figure 4).

### Example 6: Preserved Citrate Buffered 0.06% (w/v), 0.08% (w/v), and 0.125% (w/v)

### Apraclonidine Compositions

The compositions shown in Table 8 were prepared.

**Table 8**

| Ingredient | Composition A % (w/v) | Composition B % (w/v) | Composition C % (w/v) |
|---|---|---|---|
| Apraclonidine (free base) | 0.06* | 0.08* | 0.125* |
| Hypromellose 2910 (E4M) | 0.3 | 0.3 | 0.3 |
| Sodium Citrate (Dihydrate) | 0.45 | 0.45 | 0.45 |
| Citric Acid (Monohydrate) | 0.0067 | 0.0067 | 0.0067 |
| Sodium Chloride | 0.74 | 0.74 | 0.74 |
| Benzalkonium Chloride | 0.005 | 0.005 | 0.005 |
| Hydrochloric Acid | Adjust to pH 6.8 | Adjust to pH 6.8 | Adjust to pH 6.8 |
| Sodium Hydroxide | Adjust to pH 6.8 | Adjust to pH 6.8 | Adjust to pH 6.8 |
| Purified Water | QS to 100 | QS to 100 | QS to 100 |

| | | | |
|---|---|---|---|
| *added as 0.069% (w/v) apraclonidine HCl equivalent to 0.06% (w/v) apraclonidine as free base 0.092% (w/v) apraclonidine HCl equivalent to 0.08% (w/v) apraclonidine as free base 0.144% (w/v) apraclonidine HCl equivalent to 0.125% (w/v) apraclonidine as free base | | | |

### Example 7: Stability of 0.06% and 0.2% Apraclonidine Compositions

The compositions shown in Table 9 were prepared and subjected to stability testing.

**Table 9**

| Ingredient | Composition D % (w/v) | Composition E % (w/v) | Vehicle % (w/v) |
|---|---|---|---|
| Apraclonidine HCl | 0.06* | 0.2* | - |
| Hypromellose 2910 (E4M) | 0.3 | 0.3 | 0.3 |
| Sodium Citrate (Dihydrate) | 0.45 | 0.45 | 0.45 |
| Citric Acid (Monohydrate) | 0.0067 | 0.0067 | 0.0067 |
| Sodium Chloride | 0.74 | 0.74 | 0.74 |
| Hydrochloric Acid | Adjust to pH 6.8 | Adjust to pH 6.8 | Adjust to pH 6.8 |
| Sodium Hydroxide | Adjust to pH 6.8 | Adjust to pH 6.8 | Adjust to pH 6.8 |
| Purified Water | QS to 100 | QS to 100 | QS to 100 |

| | | | |
|---|---|---|---|
| *0.069 % (w/v) apraclonidine HCl added as equivalent to 0.06% (w/v) apraclonidine as free base; 0.23% (w/v) apraclonidine HCl added as equivalent to 0.2% (w/v) apraclonidine as free base | | | |

Compositions D, E, and Vehicle were packaged in opaque, white LDPE bottles and stored under one of three stability conditions:
Long-term: 5 °C ± 3 °C (simulates controlled refrigerated conditions)
Long-term: 25 °C ± 2°C / 40% RH ± 5% RH (simulates controlled ambient conditions)
Accelerated: 40°C ± 2°C / 20% RH ± 5% RH (simulates stressed storage conditions)

The testing schedule in months for the specified storage conditions is given in Table 10.

**Table 10**

| Storage Conditions | Time Point (month) / Test Requirements | | | | | |
|---|---|---|---|---|---|---|
| | Initial | 1 | 3 | 6 | 9 | 12 |
| 5 °C ± 3 °C | n/a | X | XZ | XZ | XZ | XZ |
| 25 °C ± 2°C / 40% RH ± 5% RH | n/a | X | XZ | XYZ | XZ | XYZ |
| 40°C ± 2°C / 20% RH ± 5% RH | n/a | X | XZ | XZ | NT | NT |

| | | | | | | |
|---|---|---|---|---|---|---|
| X: Chemical (assay, ID, and impurity) and Physical (appearance, pH, viscosity, osmolality, and weight change) testing Y: Sterility testing Z: Particulate Matter by HIAC NT: Not Tested | | | | | | |

The stability testing results are shown in Table 11.

**Table 11**

| **Test Attributes** | **Shelf Acceptance Criteria** | **Initial (Time Zero)** | | **Range of Results** | |
|---|---|---|---|---|---|
| - | - | - | 5°C | 25°C/40%RH | 40°C/20% RH |
| Appearance - Clarity | NMT PH.Eur. III | NMT PH.Eur. II | NMT PH.Eur. II | NMT PH.Eur. II | NMT PH.Eur. II |
| Appearance - Color by Visual Fxamination | Light Yellow, ≤ Ph.Eur.Y3 | ≤ Ph.Eur.B9 (Colorless) | ≤ Ph.Eur.B9 (Colorless) | ≤ Ph.Eur.B9 (Colorless) | ≤ Ph.Eur.B9 (Colorless) |
| API ID by HPLC Retention Time | Positive¹ | Positive¹ | NT | NT | NT |
| Apraclonidine Assay (w/v %)² | 90 to 110%, %L | 100 - 100 | 99 - 101 | 99 - 102 | 100 - 103 |
| Apraclonidine Impurities₂ | Individual: | <LOQ | <LOQ | <LOQ | <LOQ |
| | NMT 1.0 % | <LOQ | <LOQ | <LOQ | <LOQ |
| pH | 5.8 to 7.8 | 6.7 - 6.8 | 6.6 - 6.8 | 6.6 - 6.8 | 6.6 - 6.8 |
| Viscosity (cPs) (CP42 @ 30 RPM) | < 15 cPs | 7-8 | 6-9 | 6-9 | 7-8 |
| Osmolality (mOsm/kg) | 260 - 320 mOsm/kg | 279 - 299 | 279 - 304 | 279 - 308 | 279-311 |
| | | | | | |
| Sterility | Pass USP <71> | Pass | NT | Pass | NT |
| Particulate | Pass USP <789> | Pass | Pass | Pass | Pass |
| Matter, HIAC | NMT 50 particles/mL | 6-9 | 2-13 | 3-9 | 3-9 |
| ≥ 10 µm | | 1-4 | 0-4 | 0-4 | 0-4 |
| ≥ 25 µm | NMT 5 particles/mL | 0-1 | 0-1 | 0-1 | 0-1 |
| ≥ 50 µm | | | | | |
| | NMT 2 particles/mL | | | | |
| | | | | | |
| Weight Change (percent) | - | NT | Up to (-) 0.1840 | Up to (-) 1.3220 | Up to (-) 3.7377 |

| | | | | | |
|---|---|---|---|---|---|
| LOQ: 0.05% of the HPLC working concentration. NT: Not Tested ¹Negative for the vehicle ²Not applicable for the vehicle | | | | | |

The results of the stability testing met acceptance criteria when stored for up to 12 months at 5°C and at 25°C/40% RH, and up to 6 months at 40°C/20% RH.

### Example 8: Stability of 0.125% Apraclonidine Composition

Composition C of Example 6 was subjected to stability testing for 1-month. The results are shown in Table 12.

**Table 12**

| **Test** | | **Acceptance Criteria** | **Initial (Time Zero)** | **1 Month** | | |
|---|---|---|---|---|---|---|
| | | | | **5°C** | **25°C/40% RH** | **40°C/20% RH** |
| Appearance - Clarity | | NMT PH.Eur. III | NMT PH.Eur. II | NMT PH.Eur. II | NMT PH.Eur. II | NMT PH.Eur. II |
| Appearance - Color by Visual Fxamination | | Pale Yellow, ≤ Ph.Eur.Y3 | ≤ Ph.Eur.B9 (Colorless) | ≤ Ph.Eur.B9 (Colorless) | ≤ Ph.Eur.B9 (Colorless) | ≤ Ph.Eur.B9 (Colorless) |
| Apraclonidine HCl, ID by HPLC Retention Time | | Positive | Conforms | Conforms | Conforms | Conforms |
| | | | Conforms | Conforms | Conforms | Conforms |
| Apraclonidine Assay (w/v %) | | 90 - 110% of Label | 102 | 100 | 100 | 100 |
| | | | 102 | 100 | 100 | 100 |
| Apraclonidine Impurities | | Each Individual Impurity : NMT 1.0 % | Not Detected | < LOQ | < LOQ | < LOQ |
| | | Total Impurities: NMT 2.0% | Not Detected | < LOQ | < LOQ | < LOQ |
| Benzalkonium Chloride Identity | | Positive | Conforms | Conforms | Conforms | Conforms |
| | | | Conforms | Conforms | Conforms | Conforms |
| **Benzalkonium** Chloride Assay | | 80 - 120% of Label | 96 | 96 | 97 | 95 |
| | | | 97 | 97 | 99 | 97 |
| pH | | 5.8 - 7.8 | 6.6 | 6.7 | 6.7 | 6.7 |
| Viscosity (cPs) (CP42 @ 60 RPM) | | < 15 cPs | 7 | 6 | 6 | 6 |
| Osmolality | | 250-340 (mOsm/kg) | 292 | 289 | 291 | 293 |
| | | Pass USP <789> | | | | |
| | | NMT 50 particles/mL | | | | |
| Particulate Matter, | | | | | | |
| HIAC | | NMT 5 particles/mL | | NT | NT | NT |
| | ≥ 10 µm | | 1 | | | |
| | ≥ 25 µm | NMT 2 particles/mL | 0 | | | |
| | ≥ 50 µm | | 0 | | | |
| Weight Change (%) | | - | N/A | 0 | -0.1 | -0.6 |

| | | | | | | |
|---|---|---|---|---|---|---|
| LOQ: 0.05% NT: Not Tested N/A: not applicable | | | | | | |

### Example 9: Ocular Bioavailability of 0.2% Apraclonidine Aqueous Pharmaceutical Compositions

The composition shown in Table 13 was prepared.

**Table 13**

| Ingredient | % (w/v) |
|---|---|
| Apraclonidine HCl | 0.23 * |
| Hypromellose 2910 (E4M) | 0.3 |
| Sodium Citrate (Dihydrate) | 0.45 |
| Citric Acid (Monohydrate) | 0.0067 |
| Sodium Chloride | 0.74 |
| Benzalkonium Chloride | 0.005 |
| Hydrochloric Acid | Adjust to pH 6.8 |
| Sodium Hydroxide | Adjust to pH 6.8 |
| Purified Water | QS to 100 |

| | |
|---|---|
| *equivalent to 0.2% (w/v) as free base | |

A single topical ocular 40µL dose of the composition shown in Table 13 was administered via a calibrated micropipette to both eyes (OU) of 30 rabbits, tissue samples collected at 0.5, 1, 2, 4, and 8 hours following the single dose, and the amount of apraclonidine in each of the tissue samples determined using HPLC. The following tissue samples were collected and analyzed: aqueous humor, bulbar conjunctiva, vitreous humor, lens, iris ciliary body, and cornea. The results are shown in Fig. 5. These results show a large amount of the administered drug is distributed rapidly to the conjunctiva, which is the target tissue type for redness reduction.

### Example 10: Human Clinical Study to Assess Safety and Efficacy

Preservative-free versions of Compositions A, B, and C from Example 6 were prepared, and their safety and efficacy were assessed in a human clinical trial. These preservative-free versions simply omitted the benzalkonium chloride ingredient but were otherwise identical to Compositions A, B, and C. The clinical study was designed and conducted as a multi-center, double-masked, randomized, vehicle-controlled, parallel group study in 112 subjects with a history of ocular redness. The study involved 5 office visits and 2 telephone safety checks over a period of approximately 5 to 6 weeks. Eligible subjects were randomized 1:1:1:1 to receive the preservative-free versions of Compositions A, B, C or vehicle. Subjects received one drop of the assigned study article in each eye once on Day 1, followed by one drop in each eye four times daily (QID) from Day 2 - Day 28. The primary efficacy measure was investigator-assessed ocular redness on Day 1 at 8 hours post-installation. The 112 randomized subjects resulted in 29 subjects receiving the preservative-free version of Composition A, 28 subjects receiving the preservative-free version of Composition B, 28 subjects receiving the preservative-free version of Composition C, and 27 subjects receiving the preservative-free vehicle. All of the treatment groups had similar baseline disease characteristics for both efficacy and safety assessments.

### Safety Results

Based upon a review of adverse events as well as ocular and systemic safety parameters, no safety issues were observed that would preclude further clinical development of the tested drug compositions for redness relief. All three tested drug compositions were well-tolerated as administered QID for up to 1 month. Drop comfort was graded as comfortable across all eyes and treatment groups and remained relatively stable throughout the visit time points and study visits. All ocular TEAEs were reported as treatment-related and classified as mild in severity. No subject experienced ocular TEAEs classified as moderate or severe. The most common ocular treatment-related TEAE reported was the eye disorders of eye irritation, reported by 1.8% of all subjects (1 subject [3.4%] in the 0.06% group, and 1 subject [3.6%] in the 0.08% group). No other ocular treatment-related TEAE was reported in more than 1 subject.

### Efficacy Results

The primary efficacy endpoint, investigator-assessed ocular redness on Day 1 at 8 hours (480 min.) after investigational drug instillation was met. The mean post-instillation ocular redness scores on Day 1 were significantly lower for all three tested drug compositions (0.06%, 0.08%, and 0.125%) when compared to the vehicle group: 0.06% vs. vehicle, (LS Mean difference [SE] = -0.81 [0.120], 95% CI: [-1.05, -0.57], p<0.0001); 0.08% vs. vehicle, (LS Mean difference [SE] = -0.61 [0.121], 95% CI: [-0.85, -0.37], p<0.0001); and 0.125% vs. Vehicle: (LS Mean difference [SE] = -0.91 [0.121], 95% CI: [-1.15, -0.67], p<0.0001), hence demonstrating superiority in relieving redness of the eye due to minor eye irritations.

## Claims

1. An aqueous topical ophthalmic pharmaceutical composition comprising apraclonidine and a polymer, wherein the apraclonidine is present at greater than 0.05% (w/v) to no more than 0.2% (w/v).

2. The pharmaceutical composition of claim 1, wherein the apraclonidine is present at 0.06% (w/v) to 0.2% (w/v).

3. The pharmaceutical composition of claim 2, wherein the apraclonidine is present at 0.07% (w/v) to 0.15% (w/v).

4. The pharmaceutical composition of claim 3, wherein the apraclonidine is present at 0.08% (w/v) to 0.125% (w/v).

5. The pharmaceutical composition of claim **1,** wherein the apraclonidine is present at 0.125% (w/v).

6. The pharmaceutical composition of claim 1, wherein the polymer is selected from the group consisting of methylcellulose, hydroxypropyl methylcellulose, hydroxyethyl-cellulose, carboxymethylcellulose, hydroxypropylcellulose, polyvinylpyrrolidone, polyvinyl alcohol, xanthan gum, carbopol, hyaluronic acid, and hydroxypropyl guar.

7. The pharmaceutical composition of claim 6, wherein the polymer is hydroxypropyl methylcellulose.

8. The pharmaceutical composition of claim 7, wherein the polymer is present at 0.3% (w/v).

9. The pharmaceutical composition of claim 1, further comprising an ophthalmically acceptable buffer.

10. The pharmaceutical composition of claim 9, wherein the pharmaceutical composition comprises citric acid and sodium citrate.

11. The pharmaceutical composition of claim 1, further comprising a tonicity adjusting agent in an amount sufficient to cause the pharmaceutical composition to have an osmolarity of 260 to 330 mOsm/kg based on the total weight of the pharmaceutical composition.

12. The pharmaceutical composition of claim 11, wherein the tonicity adjusting agent is sodium chloride.

13. The pharmaceutical composition of claim 1, wherein the pharmaceutical composition has a pH value of 5.8 - 7.8.

14. The pharmaceutical composition of claim 1, further comprising benzalkonium chloride.

15. The pharmaceutical composition of claim 14, wherein the pharmaceutical composition comprises 0.005% (w/v) benzalkonium chloride.

16. The pharmaceutical composition according to claim 1 for use in a method of reducing ocular redness in an eye of a subject.

17. The pharmaceutical composition for use according to claim 16, wherein the pharmaceutical composition comprises apraclonidine in the form of apraclonidine hydrochloride, the apraclonidine hydrochloride is present in an amount equivalent to 0.08 - 0.125% (w/v) apraclonidine free base, hydroxypropyl methylcellulose in an amount of 0.05 - 0.5% (w/v), and a tonicity adjusting agent in an amount sufficient to cause the pharmaceutical composition to have an osmolarity of 260 to 330 mOsm/kg based on the total weight of the pharmaceutical composition, wherein the pharmaceutical composition has a pH 6.3 - 7.3.

18. The pharmaceutical composition for use according to claim 17, wherein the pharmaceutical composition further comprises benzalkonium chloride in an amount of 0.005% (w/v).

## Patentansprüche

1. Wässrige topische ophthalmische pharmazeutische Zusammensetzung, umfassend Apraclonidin und ein Polymer, wobei das Apraclonidin in einer Menge von mehr als 0,05 % (w/v) bis nicht mehr als 0,2 % (w/v) vorliegt.

2. Pharmazeutische Zusammensetzung nach Anspruch 1, wobei das Apraclonidin in einer Menge von 0,06 % (w/v) bis 0,2 % (w/v) vorliegt.

3. Pharmazeutische Zusammensetzung nach Anspruch 2, wobei das Apraclonidin in einer Menge von 0,07 % (w/v) bis 0,15 % (w/v) vorliegt.

4. Pharmazeutische Zusammensetzung nach Anspruch 3, wobei das Apraclonidin in einer Menge von 0,08 % (w/v) bis 0,125 % (w/v) vorliegt.

5. Pharmazeutische Zusammensetzung nach Anspruch 1, wobei das Apraclonidin in einer Menge von 0,125 % (w/v) vorliegt.

6. Pharmazeutische Zusammensetzung nach Anspruch 1, wobei das Polymer aus der aus Methylcellulose, Hydroxypropylmethylcellulose, Hydroxyethylcellulose, Carboxymethylcellulose, Hydroxypropylcellulose, Polyvinylpyrrolidon, Polyvinylalkohol, Xanthan, Carbopol, Hyaluronsäure und Hydroxypropylguar bestehenden Gruppe ausgewählt ist.

7. Pharmazeutische Zusammensetzung nach Anspruch 6, wobei das Polymer Hydroxypropylmethylcellulose ist.

8. Pharmazeutische Zusammensetzung nach Anspruch 7, wobei das Polymer in einer Menge von 0,3 % (w/v) vorliegt.

9. Pharmazeutische Zusammensetzung nach Anspruch 1, die weiterhin einen ophthalmisch unbedenklichen Puffer umfasst.

10. Pharmazeutische Zusammensetzung nach Anspruch 9, wobei die pharmazeutische Zusammensetzung Citronensäure und Natriumcitrat umfasst.

11. Pharmazeutische Zusammensetzung nach Anspruch 1, weiterhin umfassend ein Mittel zum Einstellen der Tonizität in einer Menge, die ausreicht, um der pharmazeutischen Zusammensetzung eine Osmolarität von 260 bis 330 mOsm/kg, bezogen auf das Gesamtgewicht der pharmazeutischen Zusammensetzung, zu verleihen.

12. Pharmazeutische Zusammensetzung nach Anspruch 11, wobei das Mittel zum Einstellen der Tonizität Natriumchlorid ist.

13. Pharmazeutische Zusammensetzung nach Anspruch 1, wobei die pharmazeutische Zusammensetzung einen pH-Wert von 5,8 - 7,8 aufweist.

14. Pharmazeutische Zusammensetzung nach Anspruch 1, die weiterhin Benzalkoniumchlorid umfasst.

15. Pharmazeutische Zusammensetzung nach Anspruch 14, wobei die pharmazeutische Zusammensetzung 0,005 % (w/v) Benzalkoniumchlorid umfasst.

16. Pharmazeutische Zusammensetzung nach Anspruch 1 zur Verwendung in einem Verfahren zur Verringerung von Augenrötung im Auge eines Subjekts.

17. Pharmazeutische Zusammensetzung zur Verwendung nach Anspruch 16, wobei die pharmazeutische Zusammensetzung Apraclonidin in Form von Apraclonidinhydrochlorid umfasst, das Apraclonidinhydrochlorid in einer Menge entsprechend 0,08 - 0,125 % (w/v) freier Base von Apraclonidin, Hydroxypropylmethylcellulose in einer Menge von 0,05 - 0,5 % (w/v) und ein Mittel zur Einstellung der Tonizität in einer Menge, die ausreicht, um der pharmazeutischen Zusammensetzung eine Osmolarität von 260 bis 330 mOsm/kg, bezogen auf das Gesamtgewicht der pharmazeutischen Zusammensetzung, zu verleihen, vorliegt, bezogen auf das Gesamtgewicht der pharmazeutischen Zusammensetzung, wobei die pharmazeutische Zusammensetzung einen pH-Wert von 6,3 bis 7,3 aufweist.

18. Pharmazeutische Zusammensetzung zur Verwendung nach Anspruch 17, wobei die pharmazeutische Zusammensetzung weiterhin Benzalkoniumchlorid in einer Menge von 0,005 % (w/v) umfasst.

## Revendications

1. Composition pharmaceutique ophtalmique topique aqueuse comprenant de l'apraclonidine et un polymère, l'apraclonidine étant présente à hauteur de plus de 0,05 % (p/v) à pas plus de 0,2 % (p/v).

2. Composition pharmaceutique selon la revendication 1, l'apraclonidine étant présente à hauteur de 0,06 % (p/v) à 0,2 % (p/v).

3. Composition pharmaceutique selon la revendication 2, l'apraclonidine étant présente à hauteur de 0,07 % (p/v) à 0,15 % (p/v).

4. Composition pharmaceutique selon la revendication 3, l'apraclonidine étant présente à hauteur de 0,08 % (p/v) à 0,125 % (p/v).

5. Composition pharmaceutique selon la revendication 1, l'apraclonidine étant présente à hauteur de 0,125 % (p/v).

6. Composition pharmaceutique selon la revendication 1, le polymère étant choisi dans le groupe constitué par la méthylcellulose, l'hydroxypropylméthylcellulose, l'hydroxyéthylcellulose, la carboxyméthylcellulose, l'hydroxypropylcellulose, la polyvinylpyrrolidone, le poly(alcool vinylique), la gomme xanthane, le carbopol, l'acide hyaluronique et le guar hydroxypropylique.

7. Composition pharmaceutique selon la revendication 6, le polymère étant l'hydroxypropylméthylcellulose.

8. Composition pharmaceutique selon la revendication 7, le polymère étant présent à hauteur de 0,3 % (p/v).

9. Composition pharmaceutique selon la revendication 1, comprenant en outre un tampon acceptable du point de vue ophtalmique.

10. Composition pharmaceutique selon la revendication 9, la composition pharmaceutique comprenant de l'acide citrique et du citrate de sodium.

11. Composition pharmaceutique selon la revendication 1, comprenant en outre un agent d'ajustement de la tonicité en une quantité suffisante pour amener la composition pharmaceutique à avoir une osmolarité de 260 à 330 mOsm/kg par rapport au poids total de la composition pharmaceutique.

12. Composition pharmaceutique selon la revendication 11, l'agent d'ajustement de la tonicité étant le chlorure de sodium.

13. Composition pharmaceutique selon la revendication 1, la composition pharmaceutique ayant une valeur de pH de 5,8 à 7,8.

14. Composition pharmaceutique selon la revendication 1, comprenant en outre du chlorure de benzalkonium.

15. Composition pharmaceutique selon la revendication 14, la composition pharmaceutique comprenant 0,005 % (p/v) de chlorure de benzalkonium.

16. Composition pharmaceutique selon la revendication 1 destinée à être utilisée dans une méthode de réduction de la rougeur oculaire dans un œil d'un sujet.

17. Composition pharmaceutique destinée à être utilisée selon la revendication 16, la composition pharmaceutique comprenant de l'apraclonidine sous la forme de chlorhydrate d'apraclonidine, le chlorhydrate d'apraclonidine étant présent en une quantité équivalente à 0,08 à 0,125 % (p/v) de la base libre de l'apraclonidine, de l'hydroxypropylméthylcellulose en une quantité de 0,05 à 0,5 % (p/v) et un agent d'ajustement de la tonicité en une quantité suffisante pour amener la composition pharmaceutique à avoir une osmolarité de 260 à 330 mOsm/kg par rapport au poids total de la composition pharmaceutique, la composition pharmaceutique ayant un pH de 6,3 à 7,3.

18. Composition pharmaceutique destinée à être utilisée selon la revendication 17, la composition pharmaceutique comprenant en outre du chlorure de benzalkonium en une quantité de 0,005 % (p/v).
